# EUROPEAN PATENT APPLICATION

(11) **EP 2 617 359 A1**
(43) Date of publication of application: **24.07.2013**
(21) Application number: 13151685.8
(22) Date of filing: 17.01.2013
(51) Int. Cl.: A61B 6/00, A61B 6/08, A61B 6/14, G01S 5/02, H05G 1/06

(54) **Alignment systems**

(30) Priority: 17.01.2012 US 201261587268 P
(71) Applicant: Aribex, Inc., Orem, UT 84097 (US)
(72) Inventor: Nguyen, Khoi, Murrieta, CA 92563 (US); McMullen, John, Orem, UT 84057 (US)
(74) Representative: Körfer, Thomas

(57) **Abstract**

Alignment systems (105) and associated methods of using such systems to help aim an x-ray source at an x-ray detector are described in this application. The alignment systems (105) can contain an electromagnetic radiation transmitter (115) (or receiver (135)) that is associated with the x-ray detector and an electromagnetic radiation receiver (135) (or transmitter (115)) that is associated with the x-ray source. Electromagnetic radiation produced by the electromagnetic radiation transmitter (115) can be detected by the receiver (135) and used to align the x-ray source and the x-ray detector in the x-ray device. In some configurations, the electromagnetic radiation transmitter (115) contains a radio-frequency (RF) antenna (or antenna array) that is attached in a fixed position relative to the x-ray detector and the electromagnetic radiation receiver (135) also contains an RF antenna (or antenna array) that is in a fixed position relative to the x-ray detector. Other embodiments are described.

## Description

### CROSS-REFERENCE TO RELATED APPLICATION

This application claims priority of U.S. Provisional Application Serial No. 61/587,268 filed January 17, 2012, the entire disclosure of which is incorporated herein by reference.

### FIELD

This application generally relates to alignment systems. In particular, this application relates to alignment systems for x-ray devices that help position an x-ray source so that it is aimed at an x-ray detector.

### BACKGROUND

Alignment systems have been used for many types of alignment. One of their uses has been to assist an operator of an x-ray device to align an x-ray source with an x-ray detector in the device. These have been especially useful in x-ray devices that are used in dental radiography.

In film based dental radiography, a cartridge containing a radiographic film is placed in the patient's mouth, (i.e., behind a patient's tooth), and an x-ray beam is projected through the tooth and onto the film. The film is developed in a dark room or a closed processor using special chemicals to obtain a radiographic image of the tooth. In filmless dental radiography, an x-ray beam is projected through the patient's tooth, but instead an electronic sensor is placed in the patient's mouth behind the tooth to be examined. The electronic sensor may include a charge-coupled device (CCD), a complementary metal oxide semi-conductor (CMOS), or any other filmless radiation sensor. The x-rays pass through the tooth and impinge on the electronic sensor, which converts the x-rays into an electrical signal. The electrical signal is often transmitted to a computer, either directly or through a module containing intermediate processing circuitry. The computer then processes the signal to produce an image on an associated output device, such as a monitor or a printer.

When a dentist or dental technician takes an x-ray of a patient's tooth, though, the x-ray source and the electromagnetic sensor (or x-ray detector) are often not properly aligned, thereby causing geometric distortion of the radiographic image and potential loss of diagnostic value. In addition, if the imaging system is misaligned there is a possibility that the area of interest is missed in the image, and the dentist or technician may need to repeat the x-ray imaging, causing unnecessary additional radiation exposure to the patient.

### SUMMARY

This application relates to alignment systems and associated methods of using such systems to help aim an x-ray source at an x-ray detector. The alignment systems can contain an electromagnetic radiation transmitter (or receiver) that is associated with the x-ray detector and an electromagnetic radiation receiver (or transmitter) that is associated with the x-ray source. The electromagnetic radiation produced by the electromagnetic radiation transmitter can be detected by the receiver and used to align the x-ray source and the x-ray detector in the x-ray device. In some configurations, the electromagnetic radiation transmitter contains a radio-frequency (RF) antenna (or antenna array) that is attached in a fixed position relative to the x-ray detector and the electromagnetic radiation receiver also contains an RF antenna (or antenna array) that is in a fixed position relative to the x-ray source.

### BRIEF DESCRIPTION OF THE DRAWINGS

The following description of the alignment systems can be understood in light of the Figures, in which:

Fig. 1 shows some embodiments of an alignment system;

Fig. 2 shows some embodiments of an electromagnetic coil that can be used in an alignment system;

Fig. 3 shows other embodiments of an alignment system;

Fig. 4 shows some embodiments of an alignment system using RF antenna as part of the electromagnetic transmitter;

Fig. 5 shows other embodiments of an alignment system using RF antenna as part of the electromagnetic transmitter;

Fig. 6 shows some embodiments of an alignment system using RF antenna as part of the electromagnetic receiver;

Figs. 7-9 show various x-ray devices with which the alignment system may be used; and

Figs. 10-11 show some methods of associating an RF transmitter with an x-ray detector.

The Figures illustrate specific aspects of the described systems and methods for alignment systems and methods for using such systems. Together with the following description, the Figures demonstrate and explain the principles of the methods and structures produced through these methods. In the drawings, the thickness of layers and regions are exaggerated for clarity. The same reference numerals in different drawings represent the same element, and thus their descriptions will not be repeated. As the terms on, attached to, or coupled to are used herein, one object (e.g., a material, a layer, a substrate, etc.) can be on, attached to, or coupled to another object regardless of whether the one object is directly on, attached, or coupled to the other object or there are one or more intervening objects between the one object and the other object. Also, directions (e.g., above, below, top, bottom, side, up, down, under, over, upper, lower, horizontal, vertical, "x," "y," "z," etc.), if provided, are relative and provided solely by way of example and for ease of illustration and discussion and not by way of limitation. In addition, where reference is made to a list of elements (e.g., elements a, b, c), such reference is intended to include any one of the listed elements by itself, any combination of less than all of the listed elements, and/or a combination of all of the listed elements.

### DETAILED DESCRIPTION

The following description provides specific details in order to provide a thorough understanding. The skilled artisan, however, would understand that the x-ray devices can be practiced without employing these specific details. Indeed, the x-ray devices can be practiced by modifying the description herein and can be used in conjunction with apparatus and techniques conventionally used in the industry. While the devices are described for alignment in x-ray imaging for dental purposes, they could be used in medical applications such as ultrasound imaging, determination of the location of catheters inside the body, or accurate positioning of surgical instruments where visual indication of the location of the instrument is obstructed by body tissue or other obstructions. As well, these alignment systems could be used for alignment in other industries, such as determination of locations of pipes inside walls in the construction industry or to determine the location and motion of a free-floating stylus or pointing device.

The alignment systems and methods for using the same in dental radiography are described herein and illustrated in the Figures. Generally, the alignment systems contain an electromagnetic transmitter (sometimes referred to as an electromagnetic generator) and an electromagnetic receiver. The electromagnetic transmitter and electromagnetic receiver are associated with the components of a device which need to be aligned (such as an x-ray source and an x-ray detector of an x-ray device). The electromagnetic field produced by the electromagnetic transmitter and can then be detected by the electromagnetic receiver. The electromagnetic transmitter and electromagnetic receiver can be aligned with each other and this alignment can then be used to help align such components of that device (i.e., the x-ray source and the x-ray detector in the x-ray device). Using multiple transmitters and/or receivers, the relative location of the transmitter(s) to the receiver(s) can be determined and the information relayed to the operator of the device.

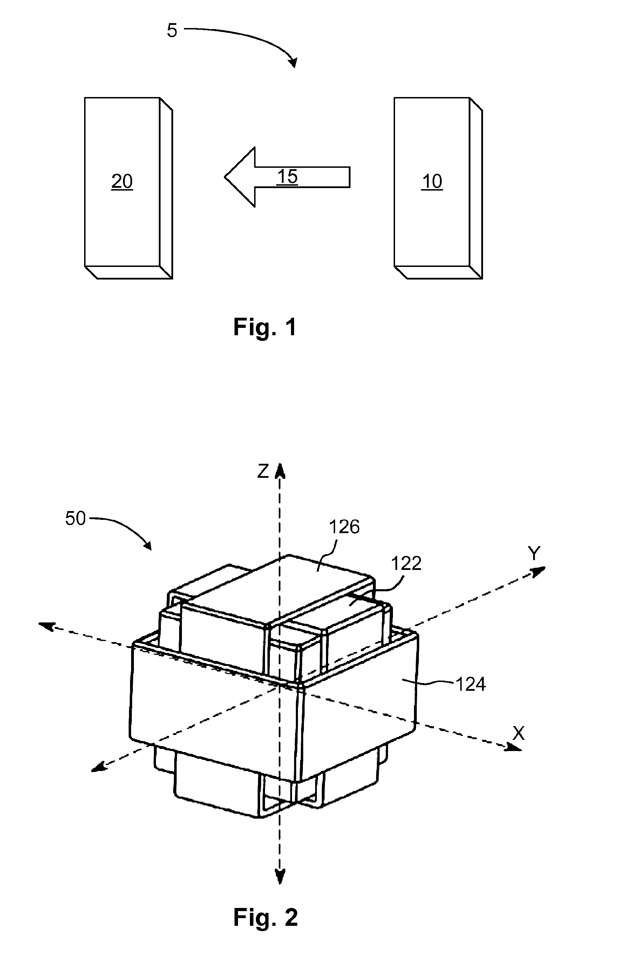

One example of an alignment system is illustrated in Figure 1. In this Figure, the alignment system 5 contains an electromagnetic transmitter 10 and an electromagnetic receiver 20. The transmitter generates electromagnetic radiation 15 and creates an electromagnetic field which is detected by the electromagnetic receiver. The type of electromagnetic radiation can be radio frequency (RF) radiation, microwave radiation, infrared radiation, or combinations thereof. In some configurations, the electromagnetic radiation comprises RF radiation. In some configurations, the alignment systems herein utilize electromagnetic waves, and not just magnetics, to operate.

In some embodiments, the electromagnetic transmitter may include one or more coils of an electrically conductive material to which a current can be applied for producing a variable electromagnetic field. The current may be modulated or may be an alternating current. The electromagnetic receiver may also contain one or more coils of an electrically conductive material for detecting the variable electromagnetic field generated by the transmitter.

The transmitter and/or receiver may also include at least two coils in different orientations for detecting at least two vector components of the variable electromagnetic field. In some configurations, the transmitter and/or receiver contains multiple sets of at least two coils, with each set of the at least two coils having a similar or the same orientation, for emitting or detecting a vector component of the variable electromagnetic field.

Figure 2 illustrates an electromagnetic coil that can be used in the alignment system 5. In Figure 2, the transmitter or receiver can contain an industry standard coil architecture (ISCA) coil packs (or assemblies) 50. In some configurations, the coil assemblies 50 may contain three approximately co-located, orthogonal quasi-dipole coils. The coil assemblies 50 may be used as transmitter coils (i.e., a transmitter coil trio) or as receiver coils (i.e., a receiver coil trio).

In some configurations, the coil assemblies 50 may contain a three-axis dipole coil transmitter or a three-axis dipole coil receiver. Each three-axis transmitter or receiver can be built so that the three coils exhibit the same effective area, are oriented orthogonally to one another, and are centered at the same point. Figure 2 is an example of a dipole coil trio coil assembly 50 with a coil 122 oriented in the X direction, a coil 124 oriented in the Y direction, and a coil 126 oriented in the Z direction. The three coils can be spaced approximately equally about a center point, as shown in Figure 2. If the coils are small enough compared to a distance between the transmitter and receiver, then the coil assembly may exhibit dipole behavior. The magnetic fields generated by the trio of transmitter coils may be detected by the trio of receiver coils. Using three approximately concentrically positioned transmitter coils and three approximately concentrically positioned receiver coils, for example, nine measurements may be obtained representing the interaction between each possible combination of receiver and transmitter coils. With the nine measurements, analytical methods can be used to solve for the six degrees of freedom that describe the receiver position and orientation with respect to the transmitter coil trio.

In these configurations, the mutual inductances between each of the three coils in the coil assembly of receiver 20 and each of the three coils in the coil assembly of the transmitter 10 can be measured. The position and orientation of the transmitter 10 with respect to the receiver 20 may then be calculated from the nine resulting mutual inductances of each of those coils and the knowledge of the coil characteristics. Thus, the position and orientation of the transmitter 10 with respect to the receiver 20 may be calculated by sensing the magnetic field generated by the transmitter 10.

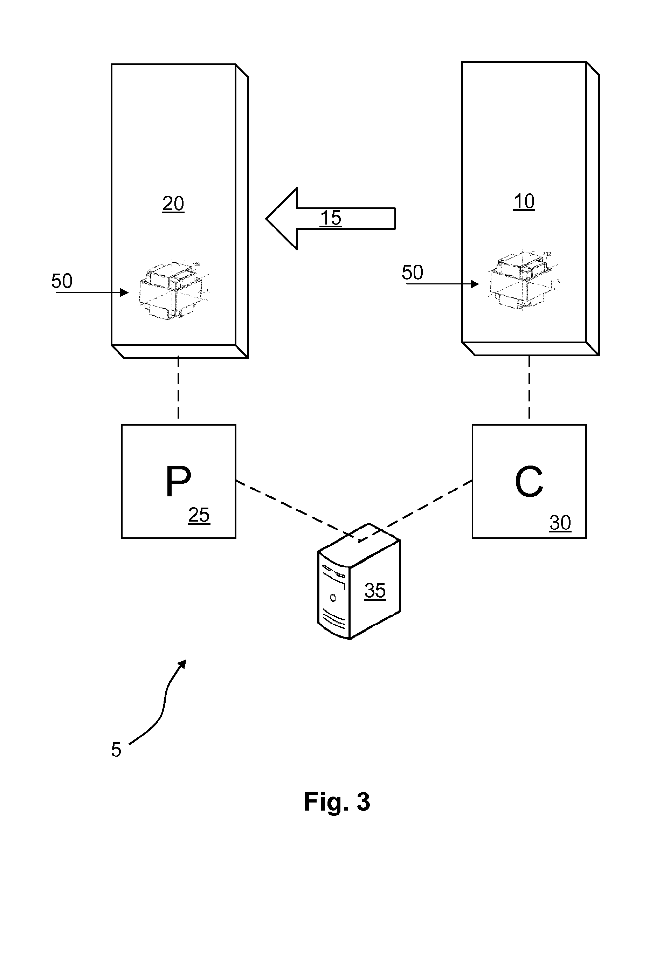

In the embodiments shown in Figure 3, the transmitter 10 and the receiver 20 can each contain a coil assembly 50. The alignment system 5 may also include a processing system 25 for determining the orientation and position of the transmitter and receiver relative to the variable electromagnetic field. In some configurations, the processing system 25 can include a band-pass filter to pass only selected frequencies of RF and therefore minimize interference from other RF signals present in the vicinity of the device. The alignment system may include a control unit 30 for controlling the transmitter 10 of the electromagnetic field. The control unit 30 and the processing system 25 may be operated so to reduce or eliminate detected electromagnetic contributions from undesired or ambient electromagnetic fields not generated by the transmitter 10. In some configurations, the control unit 30 and the processing system 25 can be connected to a computer or microcontroller 35 that can be used by an operator to operate the control unit 30 and analyze the data received from the processing system 25.

The transmitter and the receiver may be powered using any power source. In some embodiments, both the transmitter and the receiver are powered from the same source, while in other embodiments they are powered from different sources. In some configurations, the power source could be a local battery.

In other embodiments, the alignment system can contain one or more RF transmitters that contain at least one antenna and one or more RF receivers that contain at least one antenna. In these embodiments, an RF signal is produced by the RF transmitter using its antenna, producing an RF field that can be detected by the receiving antenna(s). In some configurations, the RF transmitter and/or the RF receiver can contain 4 antennas. For example, in the case of dental radiography the 4 transmitting antennas could be located at the 4 corners of a rectangular intra-oral sensor. A similar arrangement of 4 receiving antennas located at the end of the x-ray source collimator would provide the closest coupling of receivers to transmitters for the most accurate positioning. The four receiving (or transmitting) antennas can be utilized to provide relative signal strengths to a control unit that interprets the received (or transmitted) signals and provides information to the operator of the alignment system. Since the signal strength of the signal detected by the four antennas is directly proportional to the distance from the RF transmitter, it can be used to help determine the position and orientation needed for alignment. In some configurations, the antennas can be located in known positions relative to each other while being as far apart as allowed by the signal strength. In these configurations, the position accuracy can improve by adding more antennae. One receiver (or transmitter) can provide the relative position to each other, but not an overall location in 3-dimensional space. Having at least 3 antennae will provide the position in all 3 dimensions, and the accuracy improves with each additional antenna.

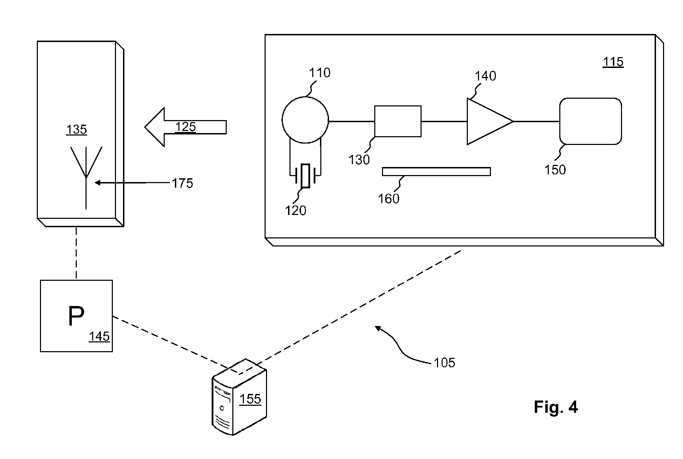

A first configuration of these embodiments is illustrated in Figure 4. In this configuration, the alignment system 105 contains an RF transmitter 115 that generates RF signal 125, and RF receiver 135. The RF receiver 135 contains one or more RF antennae 175 that receive the RF signal. The alignment system 105 also contains processing system 145 that is connected to computer or microcontroller 155.

In this configuration, the RF transmitter 115 contains a loop antennae 150, oscillator 110, frequency reference 120, filter 130, amplifier 140, and power source 160. The oscillator 110 can be used to provide the basic frequency required to generate the RF signal. Any oscillator providing the desired RF frequency can be used. The frequency reference 120 can be used to provide frequency stability under varying operating conditions of temperature and voltage. In some embodiments, the frequency reference 120/220 may comprise a crystal device, a ceramic resonator, and/or other RF generators. The frequency filter 130 removes any unwanted harmonics that could cause erroneous operation or spurious RF emissions that could interfere with other electronic equipment with which the alignment system is used. The amplifier 140 can be used to boost the RF signal to a level that will provide a sufficient RF signal that matches the impedance of the receiver antenna 150. The power source 160 can be used to provide sufficient power to operate all of these components.

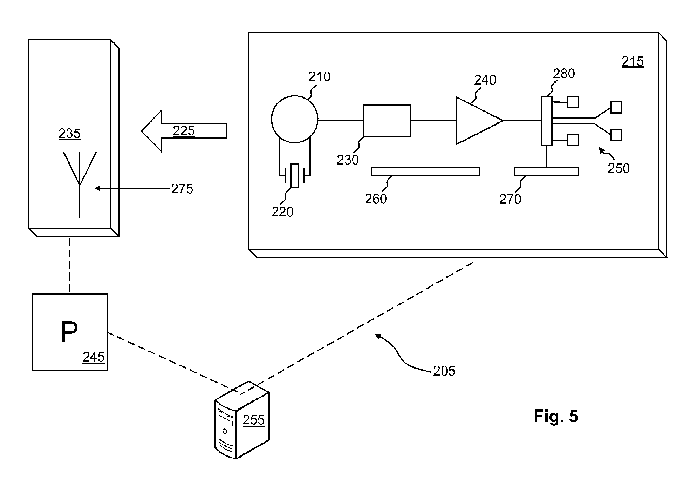

A second configuration of these embodiments is illustrated in Figure 5. In this configuration, the alignment system 205 contains an RF transmitter 215 that generates an RF signal 225 and RF receiver 235. The RF receiver contains one or more RF antennae 275 that receive the RF signal. The alignment system 205 also contains processing system 245 that is connected to computer or microcontroller 255.

In this configuration, the RF transmitter 215 contains an antenna array 250 (of up to four antennae or even more) oscillator 210, frequency reference 220, filter 230, amplifier 240, and power source 260. All of these components operate substantially similar to the same named components described with reference to Fig. 4. Because the RF transmitter 215 contains an antenna array instead of a loop antenna, an antenna distributor 280 has been added and can be utilized to switch the amplified signal to each antenna in the antenna array 250. The sequence of the switching can be controlled by an antenna sequencer 270.

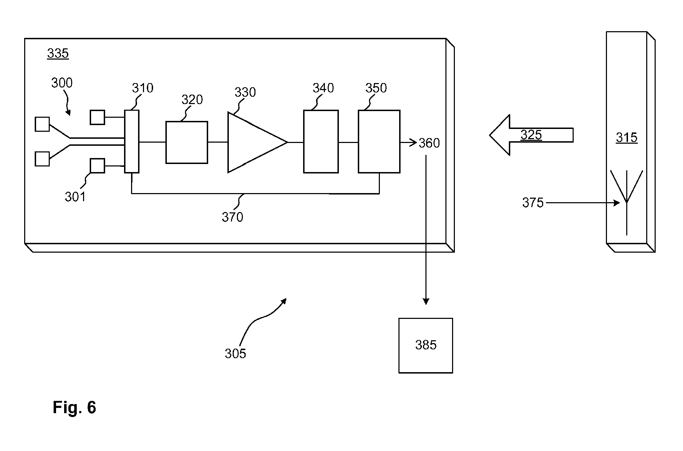

A third configuration of these embodiments is illustrated in Figure 6. In this configuration, the alignment system 305 contains an RF transmitter 315 and RF receiver 335. The RF transmitter 315 contains one or more RF antennae 375 that transmit the RF signal 325. In this configuration, the RF receiver 335 contains an antenna array 350 (of up to four or more antennae 301), antenna selector 310, filter 320, amplifier 330, power converter 340 microcontroller 350, display data 360, and display 385. As shown in Fig. 6, the four antennae 301 are spaced to form an antenna array 300. Each individual antenna 301 can be configured to detect the radio frequency signal from the transmitter 315. Each antenna 301 can be selected sequentially by the antenna selector 310 operating under the control of a microcontroller 350. As each antenna 301 is selected, the detected RF signal is passed through the filter 320 which attenuates all RF energy outside of the frequency of the transmitter 315. This filter 320 enables selective amplification of the desired RF energy while reducing potential interfering energy from other sources of RF energy in the vicinity.

The filtered radio frequency energy is then amplified. While a logarithmic amplifier can be utilized as the amplifier 330 in Fig. 6, a linear amplifier could also be utilized. The strength of the RF energy from the amplifier 330 can then be measured using a power converter 340. The output of the power converter 340 comprises a voltage level that can be measured by an analog-to-digital converter device which, as illustrated in Figure 6, comprises a microcontroller 350. The microcontroller 350 provides to selector control 370 the signals needed to control which antenna 301 is selected. As each antenna 301 is selected, the strength of the RF energy is measured. This entire process is repeated many times each second to produce display data 360 for the display 385 where a display processor calculates and displays a graphical representation of the display data 360 to the x-ray operator.

In these configurations, an algorithm can followed to calculate the position of the transmitting antenna with respect to the receiving antenna array. When utilizing the RF transmitter configuration illustrated in Fig. 4 with RF receiving antenna in Fig. 6, a total of four measurements can be produced (since there is only a single transmitting antenna and four receiving antenna). By a simple mathematical process, the distance between-and the orientation of-the single transmitting antenna with respect to the receiving antenna array 300 can be determined. When utilizing the transmitter in Fig. 5, though, a total of 16 measurements are produced since there are 4 transmitting antenna and 4 receiving antenna. This configuration allows a significant improvement in calculating the distance and orientation of the transmitting antenna array 250 with respect to the receiving antenna array 300. Each of the 4 individual antenna in the transmitting antenna array transmits energy for a short period of time for each of the four receiving antenna 301 which detect the RF energy and calculations are performed to determine the position and orientation. In other words, this calculation can be based on the diminution of the RF field strength with distance. And unlike magnetic fields, these calculations can vary based on the frequency and the medium through which the electromagnetic wave is traveling. Without being limited by this explanation, it is believed that the inverse-square law can apply since if you move twice as far away the intensity drops by a factor of four, provided absorption in the medium can be ignore (which is a good estimate at short distances used in dental radiography).

The configuration in Fig. 6 shows an antenna selector 310 being used to direct the RF energy from each antenna to the filter, the amplifier, and the energy detector. In alternative configurations, the antenna selector can be replaced multiple band-pass filters, multiple amplifiers, and multiple power converters each providing data to the microcontroller. These other configurations can provide additional sensitivity, increased accuracy, and less susceptibility to other electromagnetic energy in the vicinity.

In yet other configurations, the antenna sequencer and antenna distributor illustrated in Fig. 5 can be eliminated. These configurations would instead utilize multiple transmitters or amplifiers, each directly driving a single antenna element. This configuration would result in fewer losses in the antenna distributor. The sequencing of the transmitting antenna could then be controlled by sequencing the power to the transmitters and/or amplifiers.

The alignment systems described herein can be used with many devices that need aligning, such as ultrasound devices or x-ray devices, including those x-ray devices used in dental radiography. The alignment systems can be used with these devices by incorporating the transmitter in one portion of the device and the receiver to another location of the device or another location that contains the object to be analyzed. For example, when used to align ultrasound equipment, the transmitter (or receiver) could be attached to the ultrasound emitter that is used to analyze the patient. The receiver (or transmitter) could then be attached to a location on the opposite side of the patient. For example, in a pregnancy examination, the receiver could be located on a pad placed on the table where the patient is laying down to be examined.

When used with the x-ray devices, the electromagnetic transmitter can be associated with the x-ray detector and the electromagnetic detector can be associated with the x-ray source. Alternatively, the electromagnetic transmitter can be associated with the x-ray source and the electromagnetic detector can be associated with the x-ray detector. In some embodiments, the transmitter and/or the receiver can be associated with the x-ray detector and/or the x-ray source by being removably attached to that component. In other embodiments, the transmitter and/or the receiver can be associated with the x-ray detector and/or the x-ray source by being permanently attached to that component.

The electromagnetic field can be detected by the receiver and analyzed using the processor (or processing system) to determine the relative position and orientation of the transmitter and receiver. Since the transmitter is attached in a fixed spatial relationship to the x-ray detector and the receiver is attached in fixed spatial relationship to the x-ray source, or vice versa, the relative position and orientation of the x-ray detector and the x-ray source can be determined. Any deviation from the desired relative position and/or orientation may be corrected by moving either the x-ray source or the x-ray detector. The relative position and orientation of the x-ray detector and the x-ray source may be moved and any alignment can be corrected until a desired alignment is achieved. In this manner, once the operator has detected the correct orientation, the x-ray source can be move to maximize the measured signal strength.

When the x-ray device is used in dental radiography to take x-rays of a tooth a patient, the transmitter can be removably or permanently connected to the x-ray detector. The x-ray detector can then be placed inside the mouth of the patient, such as behind a tooth to be examined. In some configurations, to maintain the desired position in the mouth, the combination of transmitter/x-ray detector can be configured with a projecting tab on which the patient can bite to secure their position.

In those embodiments using electromagnetic coils, the position of the x-ray source and x-ray detector can be based on the acquisition of the output signals from the coils and their relevant spatial geometry. These signals can be used to reconstruct a complete model of the generated electromagnetic field and identify the relative position and orientation of the transmitter and receiver. A complete simulation of the generated electromagnetic field may be realized using finite elements analysis (FEA) tools, such as available in standard mathematic libraries, and the output signals from the detection coils. In other embodiments, the position of the x-ray source and x-ray detector can be determined based on the acquisition of the output signals from the transmitter antenna relative to the receiver antenna.

When used for dental radiography, the alignment system and/or the x-ray device may further include a display to indicate to an operator of the x-ray device any misalignment of the x-ray detector and the x-ray source. The display can indicate to an operator the appropriate directions and/or orientations to move the x-ray detector or x-ray source to achieve the desired alignment. The display can also indicate to the operator when the x-ray source is located sufficiently close to the x-ray detector so that the electromagnetic field generated by the electromagnetic transmitter is detected by the electromagnetic receiver. The display may have one or more arrays of LEDs indicating to an operator the directions or orientations to move an x-ray detector or an x-ray source to achieve the desired alignment. The display can be external to the x-ray device or integrated into the x-ray device.

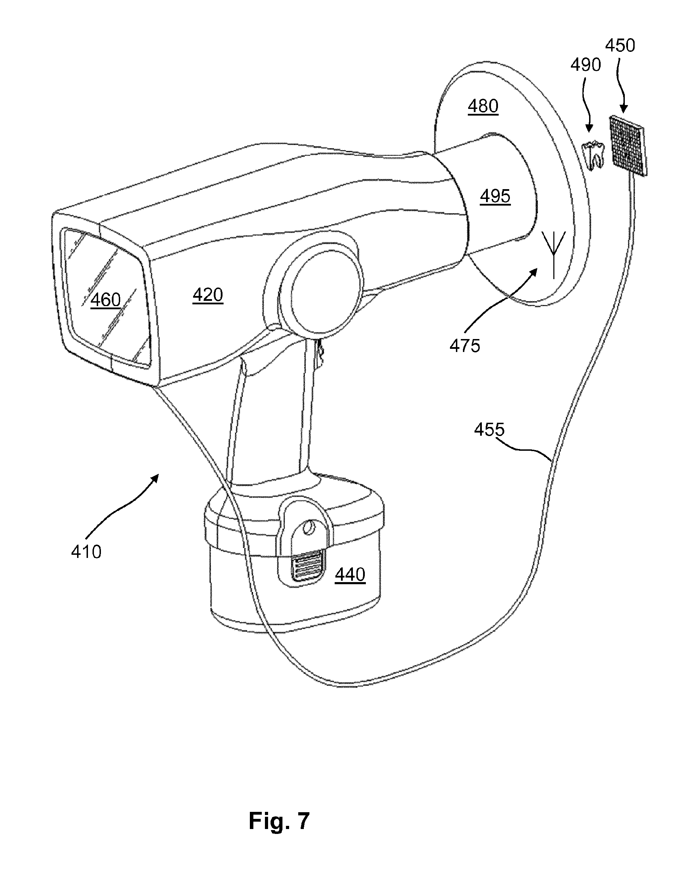

In some embodiments, the alignment system can be used with the x-ray devices shown in Figures 7-8. In these embodiments, an x-ray device 410 contains a housing or chassis 420 enclosing all the internal components of the device. The housing 420 encloses an x-ray tube which contains an x-ray source for producing the x-rays. The x-ray device 410 contains a power system (including power source 440) to provide power for the device 410 and an x-ray detector, such as film, CCD sensors, or imaging plates (not shown). The x-ray device 410 also contains a collimating cone 495 and radiation shielding 480 to shield the operator of the device from backscattered radiation. The x-ray device 410 also contains any other components for efficient operation (such as a controller, power supply, etc.).

The RF receiving antenna can be integrated into any desired location of the device 410. In some configurations, the RF receiving antenna can be incorporated into collimating cone 495. In other configurations, the RF receiving antenna can be incorporated into shielding 480 as shown in Figs 7-8. Although the antennas illustrated in Figs. 7-8 are illustrated Y-shaped, other kinds and shapers of antennas can also be used. In even other configurations, multiple RF receiving antenna can be incorporated into different components or parts of the x-ray devices shown in Figs. 7-8.

The x-ray device 410 also contains a mechanism for displaying the x-rays detected by the x-ray detector. Examples of displays that can be used include film, imaging plates, and digital image displays such as cathode ray tubes (CRT) or liquid crystal display (LCD) screens. In some configurations, and as illustrated in Figs. 7-8, the display is integrated into the housing 420 of the x-ray device. In these configurations, any small display with sufficient resolution can be used, including liquid crystal display (LCD) screen 460.

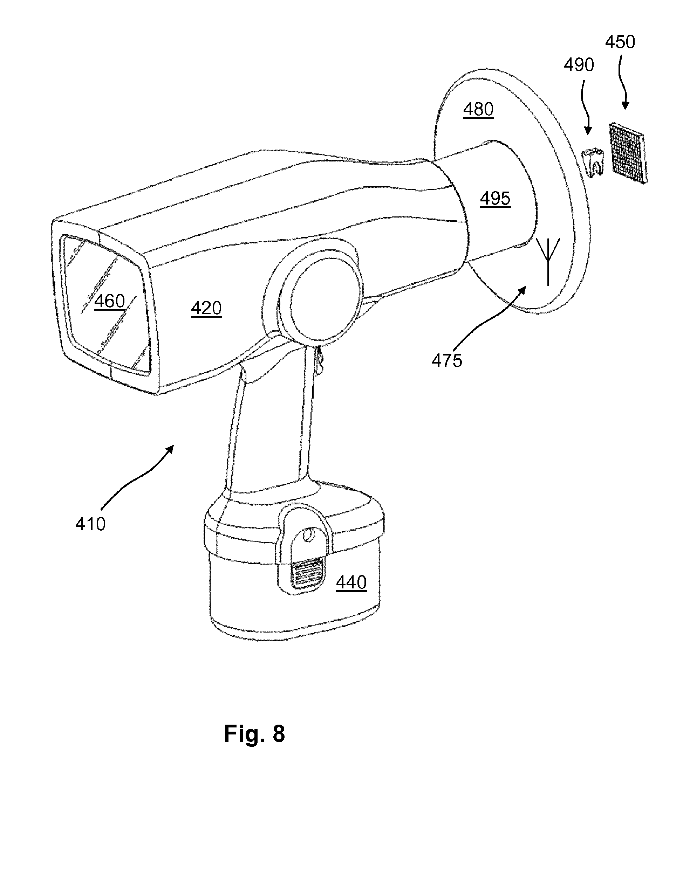

The radiographic image of the tooth 490 detected by the x-ray detector (CCD sensor 450) is transmitted to the x-ray device 410 and then viewed via the display 460. This communication can take place using a wire or a cable 455, as shown in Fig. 7. Alternatively, as shown in Fig. 8, the x-ray detector (CCD sensor 350) can communicate with the x-ray device 410 by any known wireless transmission mechanism. Examples of some wireless transmission mechanisms include 802.11 protocols, wireless application protocols (WAP), Bluetooth technology, or combinations thereof.

With such a configuration, the x-ray device 410 can be especially useful for dental radiography. The x-ray device 410 can be used to analyze a tooth 490 (or multiple teeth) of a patient by placing the tooth 490 between the x-ray device 410 and the CCD sensor 450 and then operating the device.

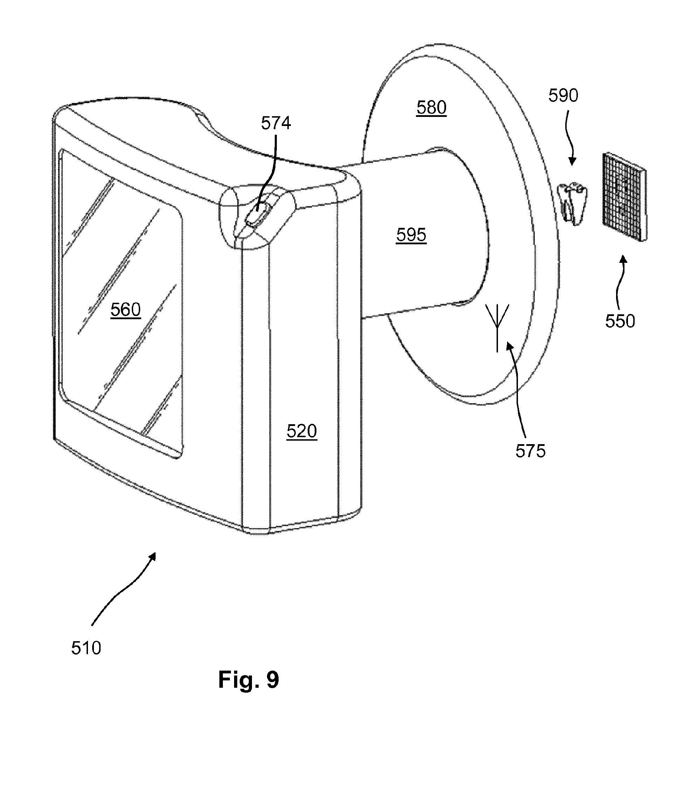

In other embodiments, the alignment systems can be used with the x-ray devices shown in Figure 9. The x-ray device 510 contains substantially the same components as-and operates substantially similar to-the x-ray device 410 but has been configured with a different shape for the housing. The housing 520 can be generally rectangular with protruding shape on the front side that provides the operator a good grip with fingers. The housing 520 can enclose an x-ray tube which contains an x-ray source for producing the x-rays that provide a radiographic image of tooth 590 using the CCD sensor 550. The x-ray device 510 contains an internal power system to provide power for the device 510 and an x-ray detector, such as film, CCD sensors, or imaging plates (not shown). The x-ray device 510 also contains a collimating cone 595 and radiation shielding 580 to shield the operator of the device from backscattered radiation. The x-ray device 510 also contains any other components for efficient operation (such as a controller, power supply, etc.).

The RF receiving antenna has been integrated into any desired location of the device 510. In some configurations, the RF receiving antenna can be incorporated into collimating cone 595. In other configurations, the RF receiving antenna can be incorporated into shielding 580 as shown in Fig. 9. Although the antennas illustrated in Figs. 9 are Y-shaped, other kinds and shapers of antennas can also be used. In even other configurations, multiple RF receiving antenna can be incorporated into different components or parts of the x-ray devices shown in Fig. 9.

In Figs. 7-9, the x-ray detector (i.e., CCD sensor) is not structurally attached to the x-ray device. Thus, the x-ray detector is free standing. Accordingly, the position of the x-ray detector illustrated in Figs. 7-9 is not fixed relative to the rest of the x-ray device, and when the x-ray detector moves, so must the -x-ray source in order to maintain a constant alignment between the x-ray source and x-ray detector. This free-standing x-ray detector in the devices of Figs. 7-9 provides an increased need to align the x-ray source and x-ray detector every time the x-ray device is used since the position of the x-ray detector can move freely relative to the rest of the x-ray device and the x-ray source. Thus, the alignment systems described herein can be especially useful where the x-ray detector is free-standing and not structurally attached.

In both types of devices illustrated in Figs. 7-9, the electromagnetic receiver (or transmitter) can be incorporated into the x-ray device (containing the x-ray source) in any location where it can sense (or transmit) the electromagnetic radiation from the transmitter (or receiver) without interfering with the x-ray emission and detection of the x-ray device. In some embodiments, the electromagnetic receiver can be integral to the device by being incorporating into the device during its manufacture. In other embodiments, the electromagnetic receiver can be removably or permanently attached to the x-ray device after it has been manufactured. In the embodiments illustrated in Figures 7-9, the electromagnetic receiver comprises an RF antenna or antenna array that has been incorporated into the respective backscatter shields of the device.

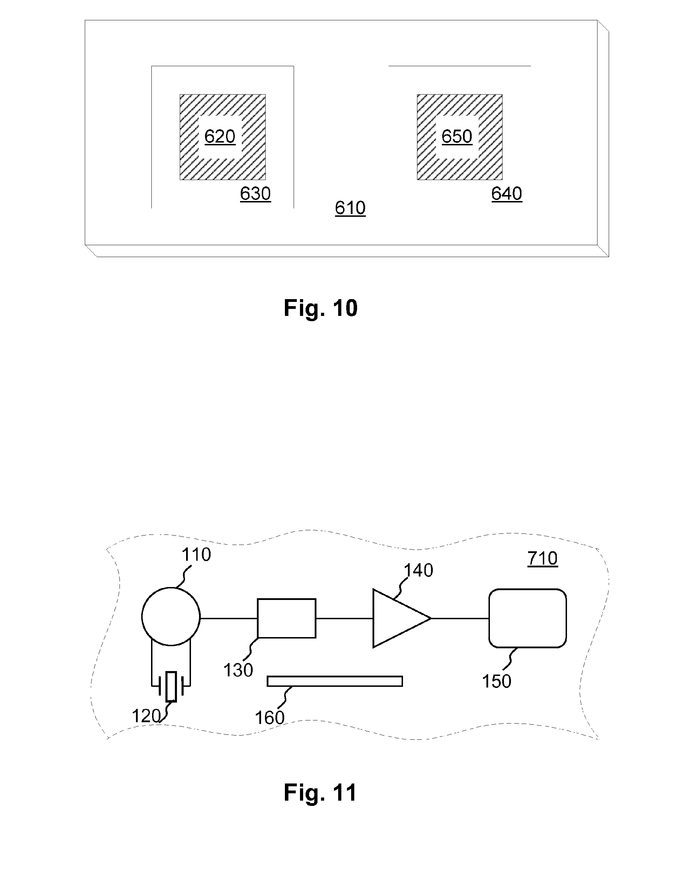

In the devices illustrated in Figs. 7-9, the electromagnetic transmitter (or receiver) can be associated with the x-ray detector in any location where it can transmit (or receive) the electromagnetic radiation without interfering with the x-ray emission and detection of the x-ray device. In some embodiments, the electromagnetic transmitter can be incorporated into a rigid housing that is configured to also contain the x-ray detector. An example of these embodiments is shown in Figure 10 where a housing 610 is configured to contain both the RF transmitter 620 in a first recess 630 and a CCD sensor 650 in a second recess 640. The housing 610 can be configured so that it may be placed inside the mouth of a patient. In some configurations, the housing 610 has an approximately rectangular shape with rounded edges, optionally containing a tab that a patient may place between teeth and used hold the housing 610 in place. When either the CCD sensor 650 or the RF transmitter 620 needs to be replaced, they can be easily removed from their respective recesses. The configuration of the housing 610 keeps the CCD sensor and the RF antenna in a substantially fixed spatial relationship. The housing 610 can be used repeatedly over and over again and so can be considered non-disposable.

In other embodiments, the housing 610 can be replaced with a disposable container. In these embodiments, as illustrated in Figure 11, a sleeve 710 is configured to be removably attached to the CCD sensor (not shown). In some configurations, the sleeve 710 contains one that is opened, then the sleeve 710 is slipped over the CCD sensor, and that end closed again to attach the sleeve to the CCD sensor. Once the dental radiography process is complete, the sleeve 710 can be removed and disposed of. The sleeve 710 may be a thin film that may be made of a natural or synthetic rubber, a latex material, a polythene, or any other similar material. The sleeve may optionally contain a tab that a patient may place between the teeth and used to hold the sleeve 710 (and therefore the CCD sensor) in place.

In these embodiments, the RF antenna and associated electronics described herein can be printed onto a surface of the sleeve 710 using thin film technology. In these embodiments, the alignment systems could operate at any approved frequency for low-power communications, including those in the ISM bands. Thus, the alignments systems do not interfere with other equipment and the other equipment is kept from interfering with the alignment systems.

These x-ray devices can be used for dental radiography in the following manner. The RF transmitter may be associated with the CCD sensor using either the housing 610 or the sleeve 710. The housing (or sleeve) is placed in the desired position in the mouth of the patient behind a tooth to be examined. The patient can bite on the tab to lock the housing (or sleeve) in place. Then, the x-ray handheld device shown in Figs. 7-9 containing the x-ray source and the RF receiver is manually moved near the jaw of the patient so that an x-ray beam generated by the x-ray source projects approximately towards the CCD sensor. Once the RF signal is transmitted and received, the relative position and orientation of the x-ray source and detector may be displayed on the display of the x-ray device. The operator can then change the position/orientation of the x-ray device until the desired alignment is obtained. In some instances, the x-ray device could then be operated so that the radiographic images can also be shown on the display.

In some configurations, these alignment systems could be used as a safety feature. The x-ray source could be configured so that it would not operate unless the alignment was achieved within a certain range of accuracy. Thus, the x-ray dose to the patient can be minimized by only taking the radiographic image when the desired alignment is obtained.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. An alignment system (5; 105; 205; 305) for aligning the components of a device, comprising:
an electromagnetic radiation transmitter (10; 115; 215; 315) comprising at least one transmission antenna adapted to transmit an electromagnetic signal, wherein the transmitter (10; 115; 215; 315) is configured to be attached to a first component of the device so that it remains in a fixed spatial relationship to that first component during operation of the device; and
an electromagnetic radiation receiver (20; 135; 235; 335) comprising at least one reception antenna adapted to receive the electromagnetic signal, wherein the receiver (20; 135; 235; 335) is configured to be attached to a second component of the device so that it remains in a fixed spatial relationship to the second component when that device is operated.

2. The system according to claim 1,
wherein the electromagnetic radiation transmitter (10; 115; 215; 315) comprises a radio-frequency antenna and/or
wherein the electromagnetic radiation transmitter comprises an oscillator (110; 210), a frequency reference (120; 220), a frequency filter (130; 230), and an amplifier (140; 240).

3. The system according to claim 1 or 2,
wherein the at least one transmission antenna comprises a plurality of RF antennae arranged in a spatially distributed array and/or
wherein the electromagnetic radiation transmitter (10; 115; 215; 315) comprises an antenna distributor (280) and an antenna sequencer (270).

4. The system according to any of the preceding claims 1 to 3,
wherein the electromagnetic radiation receiver (20; 135; 235; 335) comprises a radio-frequency antenna and/or
wherein the electromagnetic radiation receiver (20; 135; 235; 335) comprises a frequency filter (320) and an amplifier (330).

5. The system according to any of the preceding claims 1 to 4,
wherein the at least one receiver antenna comprises a plurality of RF antennae arranged in a spatially distributed array and/or
wherein the electromagnetic radiation receiver (20; 135; 235; 335) comprises an antenna selector (310) and a selector control (370).

6. An alignment system (5; 105; 205; 305) for an x-ray device, comprising:
an RF transmitter (10; 115; 215; 315) comprising an RF transmission antenna adapted to transmit an RF signal, wherein the RF transmitter (10; 115; 215; 315) is configured to be attached to an x-ray detector of the x-ray device so that it remains in a fixed spatial relationship to the x-ray detector when the x-ray device is operated; and
an RF receiver comprising (20; 135; 235; 335) an RF reception antenna adapted to receive the RF signal, wherein the RF receiver (20; 135; 235; 335) is configured to be attached to an x-ray source of the x-ray device so that it remains in fixed spatial relationship to the x-ray source when the x-ray device is operated.

7. The system according to claim 6,
wherein the RF transmitter (10; 115; 215; 315) further comprises an oscillator (110; 210), a frequency reference (120; 220), a frequency filter (130; 230), and an amplifier (140; 240) and/or
wherein the RF transmitter (10; 115; 215; 315) further comprises an array of spacially distributed RF antennae, an antenna distributor (280), and an antenna sequencer (270).

8. The system according to claim 6 or 7,
wherein the RF receiver (20; 135; 235; 335) further comprises a frequency filter (320) and an amplifier (330).

9. The system according to any of the claims 6 to 8,
wherein the RF receiver (20; 135; 235; 335) further comprises an array of spacially distributed RF antennae, an antenna selector (310), and a selector control (370).

10. An x-ray device, comprising:
an x-ray source;
an x-ray detector that is structurally unattached to the x-ray device; and
an alignment system (5; 105; 205; 305) containing:
an RF transmitter (10; 115; 215; 315) comprising an RF antenna adapted to transmit an RF signal, wherein the RF transmitter (10; 115; 215; 315) is attached to the x-ray detector so that it remains in a fixed spatial relationship to the x-ray detector when the x-ray device is operated; and
an RF receiver (20; 135; 235; 335) comprising an RF antenna adapted to receive the RF signal, wherein the RF receiver (20; 135; 235; 335) is attached to the x-ray source so that it remains in a fixed spatial relationship to the x-ray source when the x-ray device is operated.

11. The device according to claim 10,
wherein the RF transmitter (10; 115; 215; 315) further comprises an oscillator (110; 210), a frequency reference (120; 220), a frequency filter (130; 230), and an amplifier (140; 240).

12. The device according to claim 10 or 11,
wherein the RF transmitter (10; 115; 215; 315) further comprises an array of spacially distributed RF antennae, and antenna distributor (280), and an antenna sequencer (270).

13. The device according to any of the claims 10 to 12,
wherein the RF receiver (20; 135; 235; 335) further comprises a frequency filter (320) and an amplifier (330).

14. The device according to any of the claims 10 to 13,
wherein the RF receiver (20; 135; 235; 335) further comprises an array of spacially distributed RF antennae, an antenna selector (310), and a selector control (370).

15. A method for aligning an x-ray source and an x-ray detector, comprising:
providing an x-ray source;
providing an x-ray detector that is structurally unattached to an x-ray device containing the x-ray source; and
providing an alignment system (5; 105; 205; 305) containing:
an RF transmitter (10; 115; 215; 315) comprising an RF antenna adapted to transmit an RF signal, wherein the RF transmitter (10; 115; 215; 315) is attached to the x-ray detector so that it remains in a fixed spatial relationship to the x-ray detector when the x-ray device is operated; and
an RF receiver (20; 135; 235; 335) comprising an RF antenna adapted to receive the RF signal, wherein the RF receiver (20; 135; 235; 335) is attached to the x-ray source so that it remains in a fixed spatial
relationship to the x-ray source when the x-ray device is operated; and using the alignment system (5; 105; 205; 305) to properly align the x-ray source with the x-ray detector.
